# EUROPEAN PATENT SPECIFICATION

(11) **EP 2 986 178 B1**
(45) Date of publication and mention of the grant of the patent: **21.02.2018**
(21) Application number: 14720209.7
(22) Date of filing: 15.04.2014
(51) Int. Cl.: A43B 7/14, A41B 11/00, A61F 5/01

(54) **ORTHOTIC FOOT SUPPORT**
ORTHESE ZUR FUSSUNTERSTÜTZUNG
ORTHÈSE POUR UN SUPPORT DU PIED

(30) Priority: 16.04.2013 GB 201306884
(43) Date of publication of application: 24.02.2016
(73) Proprietor: DM Orthotics Limited, Redruth, Cornwall TR15 1SH (GB)
(72) Inventor: SAWLE, Leanne, Redruth Cornwall TR15 1SH (GB); MATTHEWS, Martin, Redruth Cornwall TR15 1SH (GB)
(74) Representative: Carridge, Andrew Edward
(86) International application number: PCT/GB2014/051175
(87) International publication number: WO 2014/170660

(56) References cited:
- WO-A2-2012/038700
- US-A- 5 460 601
- US-A- 5 617 745
- US-A- 5 865 779

## Description

This invention relates to a dynamic orthotic support, in particular to a foot support for the management of plantar fasciitis by helping to alleviate the symptoms thereof.

### Background to the invention

The plantar fascia is a band of tissue containing longitudinally-orientated collagen fibres that run under the sole of the foot, connecting the heel bone with the base of the toes. The plantar fascia contributes to the support of the arch of the foot, undergoing tension when the foot bears weight, and facilitating foot movement during gait.
Plantar fasciitis is one of the most common foot complaints in adults. Individuals with this condition frequently experience pain on the underside of their heel during weight-bearing. Plantar fasciitis occurs when the plantar fascia becomes damaged and inflamed, which can occur as a result of too much stress, or repetitive stress. Plantar fasciitis can also result from over-pronation (flat feet), in which the arch collapses during weight-bearing or if there is excessive weight on the foot, usually attributed to obesity or pregnancy. The condition can also lead to an increased probability of knee pain, especially amongst runners.
A common method to relieve the pain associated with plantar fasciitis is to apply tape, such as Kinesio® tape, to the affected foot. The tape relives pain by restricting the permitted range the plantar fascia can stretch. Although taping can be effective in certain situations, it has a number of disadvantages. For effective application, it requires a specialist, such as a physiotherapist, to apply the tape. Furthermore, during sporting activity, the tape can loosen and stretch, resulting in a decrease in its effectiveness over time and a requirement for frequent reapplication. Another common method to relieve pain associated plantar fasciitis is to apply a night splint to the affected foot to stretch the plantar fascia.

US5,460,601 discloses the features of the preamble of claim 1 and teaches the use of an elastic footwrap for treating plantar fasciitis by relaxing and shortening the musculatures of the foot.

US5,865,779 discloses an enveloping elastic sock for treating plantar fasciitis. The sock has a heel opening and exerts compressive forces along the longitudinal and transverse axes of a patient's foot.

US5,617,745 discloses a support sock that stabilises the ankle without the use of bulky bandages or specialized orthotic-type shoes. The sock has elastic material around the ankle area extending down to the arch where the arch and instep are securely bound.

WO2012/038700A2 discloses an orthotic device which has a first, resilient portion configured to apply a force in one or more predetermined directions to assist or restrict movement of at least one part of a wearer's body, and a second, rigid portion configured to restrict movement of at least one part of the wearer's body. The second portion is removably-couplable to the first portion. The orthotic device may be an ankle-foot orthosis.

It is an aim of the present invention to provide an improved orthotic support for the management of plantar fasciitis.

### Summary of the invention

The invention provides a dynamic orthotic support and a method of making an orthotic support as defined in the appended independent claims to which reference should now be made. Advantageous or preferred features are set forth in dependent claims.

In its broadest sense, the invention is directed to a dynamic orthotic support or device for use in the management of plantar fasciitis. In particular, the support may assist in alleviation of the symptoms, such as the pain, associated with the condition. The support may help in the treatment of plantar fasciitis, for example as part of a physiotherapist's management plan for a patient. In use, the support may urge the wearer's foot or parts of the wearer's foot into a particular position or orientation. As plantar fasciitis may result from a straining or overstretching of the plantar fascia, the support may help to reduce strain on the wearer's plantar fascia. The support may achieve this, at least in part, by urging the foot towards a closed position and off-loading the plantar fascia. This may involve dynamically urging the foot into a position that shortens the distance between the ball of the foot (including the base of the toes and the head of the metatarsals) and the heel (or calcaneus) of the foot. The support may increase or accentuate the height of the longitudinal arch of the foot (which may include the lateral and/or medial longitudinal arches). The support may also urge the foot to close in a lateral or transverse direction. For example, the support may urge a left part of the sole towards a right part of the sole. This action may increase the height of a transverse arch of the foot.

The invention provides a dynamic orthotic device or support for the management or treatment of plantar fasciitis comprising a sock having a sole portion formed from a resilient material. The sole portion is shaped such that, when the support is worn by a patient or wearer, lines of tension are initiated in the sole portion. These lines of tension act to urge the wearer's foot towards a closed position and thereby help to alleviate the symptoms of plantar fasciitis.

The sole portion may be said to comprise a heel section, a ball-of-foot section, and an arch section. The sole portion is preferably shaped such that, when worn, material of the arch section is stretched by a greater proportion than material of the heel section or material of the ball-of-foot section. This may initiate lines of tension in the sole portion that act to urge the wearers foot towards a closed position.

In a preferred embodiment of a support, the sock comprises a resilient material for conforming to the foot and ankle of a wearer, material at the sole portion being indented (or concave) such that, when worn, the indented portion is stretched to conform to the sole of the wearer's foot thereby initiating the lines of tension. The resilient material may be termed an underlying resilient material. Preferably, the indent (or concavity) is substantially conical when viewed in three-dimensions, such that when it is stretched to conform the wearer's foot, it initiates lines of tension that act to close the wearer's foot both longitudinally and transversely.

The indent may be formed at an indent seam that runs substantially centrally along the sole portion. The indent seam may result from a method of manufacturing the device. A method of making the device comprises the steps of forming a sock from resilient material for conforming to a wearer's foot and forming a dart in a sole portion of the sock.

A dart may be formed by gathering or pleating a portion of the material on the sole portion of the sock. This material may then be fixed or joined to form an indent into the sole, for example by sewing.

In a preferred embodiment, a dart may be formed by removing a portion of material from a sole portion of the sock to leave a hole, aperture or opening defined through the sole portion of the sock. Edges of the hole may then be joined such that a portion of the sole portion is indented relative to the remainder of the sock. The remaining parts or edges of the opening may be joined together by, for example, sewing or stitching them together. Formation of a seam may thus hold the edges of the opening together.

The term "dart" encompasses a fold or pleat sewn into a fabric to provide a three dimensional shape to the fabric as well as a region in which material has been removed to define a gap or hole, the edges of which have been joined together. A dart in the sole portion effectively forms an indent in the sole portion.

The indent seam or the dart may run or extend from the ball-of-foot section of the sole portion to the heel section of the sole portion. The indent may be arranged such that, in use, it is positioned at least partly at, or under, the longitudinal arch of the wearer's foot, which may include the lateral and/or medial longitudinal arches. The indent in the sock may thus mimic or accentuate a shape or position of the longitudinal arch of the wearer.

The indent, the indent seam, or the dart may have a length of 3 to 20 cm, for example 5 to 15 cm or 7cm to 10cm, although the length of the seam or length of the indent may vary depending on the size of the wearer's foot to which the support is designed to fit.

At its deepest point, the depth of the indent, when the support is not being worn, and is not under stress may be at least 1 cm. Preferably, the depth of the indent is from 1 to 3 cm. However, the depth of the indent may vary depending on the size of the wearer's foot of which the support is intended to fit and may, for example, be a function of the circumference of the foot and/or the length of the foot. The magnitude of the force exerted on the wearer's foot, in use, may be tailored by altering the depth of the indent. A deeper indent may generate a greater elastic force when a wearer's foot is inserted into the support.

Preferably, the support is entirely fabricated from one or more types of resilient material. A resilient material is a material that can elastically deform under load and return to its original shape when the load is removed, for example rubber materials or elastomeric fabric materials. Resilient materials that may be suitable for manufacturing a dynamic support as described herein include elastomeric materials such as Lycra®.

Preferably, the device does not have any rigid parts for supporting the plantar fascia. Preferably, the support is suitable for wearing underneath a sports sock and with sports shoes such as training shoes.

The sock portion of the support may also conform to the ankle and/or lower leg of the wearer, or at least a portion of the ankle and/or lower leg of the wearer. In some embodiments of the support, one or more fastenings such as a zip or zips may be utilised to facilitate donning of the support by the wearer.

In a preferred embodiment, the support may comprise at least one resilient reinforcement panel or strip on the sock, or attached to the sock, for urging the foot or parts of the foot into a position or orientation that reduces the symptoms of plantar fasciitis (for example, at least one resilient reinforcement may assist the closure of the foot as described above). Preferably the panel or panels are at least partially positioned on the sole portion of the sock. Preferably the panel or panels cooperate with the shape of the sole portion of the support to increase the force acting to close the foot.

The one or more strips or panels of resilient reinforcement material, for example elastic material, may be attached to a base/underlying elastomeric material forming the sock of the support. The panels may thus provide a tensile, contractile or compressive force to a portion of the wearer's foot, and possibly also the wearer's ankle and lower leg. The panels may initiate lines of tension, when worn. That is, the resilient reinforcement panels may be stretched when the support is worn, and thereby initiate an elastic force that acts to urge a wearer's foot towards a closed position.

In preferred embodiments, panels or strips of resilient material may be attached to the sock in a non-tensioned condition, i.e. the panels or strips do not have a force applied to them as they are attached to the underlying material forming the sole portion and do not exert a force on the underlying material. In this configuration each panel or strip may, when the device is worn, apply a resistive force acting in a specific direction when a portion of a wearer's foot or leg is moved in a direction that causes the panel or strip to stretch. The force generated by an individual strip or panel is preferably applied to the wearer's foot or leg in the direction of a longitudinal axis of the strip or panel. More than one strip or panel may be used in conjunction such that the sum of the forces applied by the strips or panels results in a net force that is applied to the wearer's body in a direction that is not coincident with a longitudinal axis of any one panel or strip. Advantageously, panels or strips of resilient material may be attached to the sock such that they become stretched when the support is worn. Thus, the resilient panels or strips may, once the support has been donned by a wearer, provide a continuous force urging a portion of the wearer's body, in particular a portion of their foot, in a predetermined direction.

In some embodiments, one or more strips or panels of resilient material may be attached to the sock in a pre-tensioned condition, i.e. the panels or strips are stretched and then attached to the underlying material while stretched. In this configuration the panels or strips are able to exert a force on a wearer's body that continually urges the wearer's body in the direction of the applied force. The force generated by a pre-tensioned strip or panel will resist movement of a wearer's foot in a direction that causes the strip or panel to stretch further.

Properties of the reinforcement panels, such as panel width or panel thickness or panel material, may be varied to increase or decrease the magnitude of the applied force. The position or shape of the reinforcement panels may be varied in order to vary the direction of the applied force.

Reinforcement panels may be positioned over pre-existing seams in the underlying support, for example, seams joining panels or sections that form the sock. Alternatively, seams, such as seams joining panels to form the sock may be formed over the top of reinforcement panels. In a preferred method of making the support, the reinforcement panels are attached to the sole portion of the sock prior to the step of forming a dart to define an indent.

The support may be a pre-fabricated support, reducing the need for strapping and tape. The wearer may put on the support such that reinforcements are automatically placed in the correct position to support the foot. This may reduce time that would otherwise need to be spent taping, and may be done without the help of another individual.

In addition, the support may enhance the wearer's proprioception i.e. the individual's subconscious awareness of their foot position. Thus, the wearer may be made aware of when their foot is in an optimal or sub-optimal position. For example, the shape of the sole portion may initiate tension which provides resistance and informs the wearer as to when the foot is being moved into an undesirable position.

Preferably, the sock is fabricated from a first resilient material and any resilient reinforcement panel or panels attached to the sock are formed from a second resilient material. Preferably the second resilient material has a higher elastic modulus than the first resilient material; that is the second resilient material is less elastically deformable than the first resilient material. Alternatively, the reinforcement panels may be made from the same material as the first resilient material.

Preferably, the first resilient material, which may be termed an underlying elastomeric material, is made from a resilient elastomeric material that conforms to the wearer's foot, ankle and/or lower-leg shape. The first resilient material preferably exhibits multidirectional stretch, i.e. the material is capable of conforming to the wearer's foot but does not generate significant overall forces, or give rise to lines of tension or compression, in any specific direction unless it is stretched preferentially in a specific direction. The first resilient material is preferably formed from any suitable elastomeric material, for example materials comprising a polyurethane-polyurea copolymer such as Dorlastan®, Spandex®, or Lycra ®. A particularly suitable material may be a polyamide-cotton-Dorlastan® material, for example a material comprising 51% polyamide, 17% cotton and 32% Dorlastan®.

Preferably, the second resilient material is fabricated from a material that provides less elastic deformation, or greater stiffness, than the first resilient material. Such a material may be defined as having an increased elastic modulus. This may increase the ability of the material to exert a force in a predetermined direction even when not stretched to a great degree in the predetermined direction. The second material may have anisotropic properties i.e. a material that allows a lower elastic deformation in one direction than another. A suitable material may be, for example a nylon/cotton material or a nylon/ Lycra ® material, for example a material comprising 81% polyamide and 19% Lycra®.

Reference herein to a resilient reinforcement panel that exerts a force in a pre-determined direction or that extends, or is arranged, in a particular direction, or along a particular axis, may also refer to a group of panels that act together or cooperate to exert a force in the pre-determined direction. For example, there may be two or more discrete panels arranged in parallel and/or end-to end, which combine to exert a force in the same direction, or along the line of tension.

Preferably, the support comprises one or more resilient panels on the sole portion of the sock. The panels may thus help to exert a force on the sole of the wearer's foot, in use, such as urging the foot to close. The panel or panels on the sole portion may extend from a periphery or edge of the sole portion, to a substantially central position on the sole portion. The central position may be with respect to the width and/or length of the sole portion. Any panel or panels of resilient reinforcement on the sole portion are preferably orientated diagonally, and may converge at a substantially central portion.

In one embodiment, a resilient panel, such as a first resilient panel, extends diagonally across and along at least part of the sole portion of the sock between a right side of a toe section or a ball-of-foot section on the sole portion, and a more rearward and leftward position of the sock, on the sole portion. In use, the panel may thus extend from the ball of the wearer's foot, or the wearer's toes (such as the base of the wearer's toes), or the wearer's metatarsals (such as the heads of the metatarsals) on a right side of the foot. Preferably, the panel does not extend to the front of the toes.

The more rearward and leftward position is preferably a position on the sole portion that is substantially equidistant from a right edge (or side, or periphery) and a left edge (or side, or periphery) of the sole portion. This may be at a point that is substantially central with respect to the width of the sole portion or with respect to the transverse, or lateral axis across the sole portion. A centre line or seam may run or extend longitudinally, or length-ways and substantially centrally along at least part of the sole portion. The panel may extend to this line. In an alternative embodiment, the panel may extend across and backwards to at least a left side of the heel portion of the sock.

The sole portion of the sock is the part that, in use, covers or conforms to the sole or plantar of the wearer's foot.

The heel section of the sock is the part that, in use, covers or conforms to the heel or calcaneus of the wearer's foot. Part of the heel section may thus be located on the sole portion.

The toe section of the sock is the part that, in use, covers or conforms to the wearer's toes. Part of the toe section may be located on the sole portion. However, the sock may be an open-toed sock that exposes at least one of the wearer's toes, in use. In this case, the toe section may be a part of the sock defined by an opening in the front of the sock.

The ball-of-foot section is the part that, in use, covers or conforms to the ball of the wearer's foot. Thus, it may represent a specific part of the sole portion.

The first resilient panel may be arranged to, when the support is in use, exert a force in a direction that urges a front, right part of a wearer's foot, such as the ball of the foot, backwards and/or leftwards.

The front or anterior of the sock or of the wearer's foot refers to the portion of sock or foot towards the toe-end. The back, rear or posterior of the sock or of the wearer's foot refers to the portion of sock or foot towards the heel-end. References to rearward and forward may thus refer to a position along the foot or with respect to a longitudinal axis of the foot. A rearward position may thus refer to a part of the foot that is more towards the heel-end, whereas a forward position may refer to a part of the foot that is more towards the toe-end.

In another embodiment, a resilient panel, such as a second resilient panel, extends diagonally across and along at least part of the sole portion of the sock between, a left side of a toe section or ball-of-foot section on the sole portion and a more rearward and rightward position of the sock on the sole portion. In use, the panel may thus extend from the ball of the wearer's foot, or the wearer's toes (such as the base of the wearer's toes), or the metatarsals (such as the heads of the metatarsals) on a left side of the foot. Preferably, the panel does not extend to the front of the toes.

The more rearward and rightward position is preferably a position on the sole portion that is substantially equidistant from a right edge (or side, or periphery) and a left edge (or side, or periphery) of the sole portion. This may be at a point that is substantially central with respect to the width of the sole portion or with respect to the transverse, or lateral axis across the sole portion. The panel may extend to the centre line or seam. In an alternative embodiment, the panel may extend across and backwards to at least a right side of the heel portion of the sock.

The second panel may exert a force in a direction that urges a front, left part of a wearer's foot, such as the ball of the foot, the base of the toes, or the metatarsals (such as the heads of the metatarsals) on a left side of the foot backwards and/or rightwards.

In another embodiment, a resilient panel, such as a third resilient panel, extends diagonally across and along at least part of the sole portion of the sock from, or between, a right side of the heel section on the sole portion to a more forward and leftward position on the sole portion.

The more forward and leftward position is preferably a position on the sole portion that is substantially equidistant from a right edge (or side, or periphery) and a left edge (or side, or periphery) of the sole portion. This may be at a point that is substantially central with respect to the width of the sole portion or with respect to the transverse, or lateral axis across the sole portion. The panel may extend to the centre line or seam.

The third panel may exert a force in a direction that urges a back, right part of a wearer's foot, such as the heel, forwards and/or leftwards.

In another embodiment, a resilient panel, such as a fourth resilient panel, extends diagonally across and along at least part of the sole portion of the sock from, or between, a left side of the heel section of the sole portion, to a more forward and rightward position on the sole portion.

The more forward and rightward position is preferably a position on the sole portion that is substantially equidistant from a right edge (or side, or periphery) and a left edge (or side, or periphery) of the sole portion. This may be at a point that is substantially central with respect to the width of the sole portion or with respect to the transverse, or lateral axis across the sole portion. The panel may extend to the centre line or seam.

The fourth panel may exert a force in a direction that urges a back, left part of a wearer's foot, such as the heel, forwards and/or rightwards.

Embodiments may comprise one or more of the first, second, third and fourth panels in any combination. A single panel may provide the function of any two or more of the resilient panels.

In some embodiments, first and second panels may meet, for example at a central point on the sole section. They may form a V-shape or chevron, with an apex pointing away from the front of the support or the toe section, towards the back of the support or heel section. The apex may be positioned at the longitudinal, central seam extending along at least part of the sole portion.

In some embodiments, third and fourth panels may meet, for example at a central point on the sole section. They may form a V-shape or chevron with an apex pointing towards the front of the support or toe section, away from the heel section or back of the support. The apex may be positioned at the longitudinal, central seam extending along at least part of the sole portion.

In some embodiments, first, second, third and/or fourth panels may form a cross or X-shape on the sole portion, the panels converging at a central point or section of the sole portion. The first and second panels may each extend diagonally across and along the sole portion to at least a position on the left side and right side of the heel, respectively. This may mean that the first and second panels effectively provide the same function as the third and fourth panels, rendering the third and fourth panels unnecessary.

Although the first, second, third and/or fourth panels are preferably distinct, they may be part of the same panel, and so may represent first, second, third and/or fourth parts, or sections, of a reinforced panel.

The support may comprise a resilient reinforcement panel (such as a transverse, lateral or cross panel) positioned at least partly on the sole portion of the sock and orientated in a substantially transverse or lateral direction, across the sole portion. Preferably, such a transverse panel crosses the first and/or second panels (as described in any form above). It may cross above or below the first and second panels. The transverse panel may extend across at least part of the ball-of-foot section, such that, in use, the transverse panel may be positioned against or at, the ball of the wearer's foot. Preferably, the transverse panel extends at least from a right edge, or periphery of the sole portion to a left edge, or periphery of the sole portion. The transverse panel may assist closing of the foot in a lateral direction and assist formation of the transverse arch. Preferably the transverse panel does not extend to the front of the toes, and may extend only as far as the ball of the foot, in use.

On the sole portion, the longitudinal axis of each panel (for example any of the first to fourth panels) or the direction of the force that each panel exerts may be arranged at an angle of between 20 and 70°, for example between 30 and 60° or 40 and 50°, with respect to the width of the sole portion or a lateral/transverse axis across the sole portion.

The length of each resilient reinforcement panel on the sole portion, for example the length of any of the first to fourth reinforcement panels as described above, may be from 2 to 10 centimetres (cm), most preferably 3 to 8 centimetres, although the length of the panel may depend on the size of the support or the size of the sock. The support may be available in a range of sizes to fit varying sizes of feet.

The width of each panel on the sole portion may be from 1 to 5 cm, preferably 2 to 4 cm.

The support may comprise a resilient reinforcement panel or panels that extend across the heel section on the back of the sock which may be termed a heel panel or heel panels. This may be part of the heel section that is not on the sole portion of the sock. A first heel panel may extend from a left part of the heel section on the sole portion, across the back of the heel section, to a right part of the heel section, and preferably up to an ankle section on the right side of the sock. The ankle section is the part of the support that covers or conforms to at least part of the wearer's ankle, in use. A first heel panel may thus extend diagonally across and up the heel of the wearer, in use. A first heel panel may be continuous with, or part of, the first panel or the fourth panel, as described in any form above.

Similarly, the support may comprise a second heel panel that extends from a right part of the heel section, on the sole portion, across the back of the heel section, to a left part of the heel section, and possibly up to an ankle section on the left side of the sock, to cover at least part of the left ankle of the wearer, in use. The second heel panel may thus extend diagonally across and up the heel of the wearer, in use. The second heel panel may be continuous with, or part of, the second panel or the third panel, as described in any form above.

The support may comprise a resilient reinforcement panel or panels that extend from an ankle section and/or heel section on the right side of the sock forwards along at least part of a top section and/or right side section of the sock, towards the toe section. It may be termed a right side panel. The panel may thus extend in a substantially longitudinal direction. The panel may extend from the ankle or heel section to the toe section. The panel may be continuous with, or part of, the first panel or fourth panel as described in any form above. For example, the support may comprise a panel that extends from a central seam on the sole portion, leftwards and rearwards to a left part of the heel section on the sole portion, across and up the back of the heel section to the ankle section and heel section on the right side and then forwards along the right side of the sock to the toe section.

Similarly, the support may comprise a resilient reinforcement panel or panels that extend from an ankle section and/or heel section on the left side of the sock forwards along at least part of a top section and/or left side section of the sock, towards the toe section. It may be termed a left side panel. The panel may thus extend in a substantially longitudinal direction. The panel may extend from the ankle or heel section to the toe section. The panel may be continuous with, or part of, the second panel or third panel as described in any form above. For example, the support may comprise a panel that extends from a central seam on the sole portion, rightwards and rearwards to a right part of the heel section on the sole portion, across and up the back of the heel section to the ankle section and heel section on the left side and then forwards along the left side of the sock to the toe section.

Panels on the heel section, ankle section and the side or top of the sock may act to increase compression on the foot and may act to increase the effect of panels on the sole portion of the sock. Properties of the panels, such as panel width, panel length, panel thickness and panel material, may be varied to increase or decrease the magnitude of the applied force. The properties may also vary depending on the size of the foot to which the support is designed to fit. The position or shape of the reinforcement panels may be varied in order to vary the direction of the applied force.

It is contemplated that a dynamic orthotic support may comprise an indent in the sole portion to assist in closing the foot or off-loading the plantar fascia, or the dynamic orthotic support may comprise the one or more resilient panels, as described in any form above, to assist in closing the foot or off-loading the plantar fascia. However, a dynamic orthotic support may work optimally in the management of plantar fasciitis when the indent on the sole portion of the sock is incorporated into the sock in addition to the one or more resilient reinforcement panels. In preferred embodiments a dynamic orthotic support comprises both an indent or dart that shapes a sole of the support to promote foot-closure, and resilient panels attached to the sole of the support that augment the effect of the indent or dart. For example, when a user places their foot into the support, the support may stretch to eliminate the indent and conform to the wearer's foot. Any reinforcement panels, especially those positioned at least partly on the sole portion, may be positioned or arranged around the indent, and/or at least partially within the indent. Such panels may be stretched to a greater extent than if the indent is not present and the resultant elastic or contractile forces may urge the foot into a desirable orientation. The reinforcement panels positioned at least partly on the sole portion of the sock may each have one end (or a first end) positioned at, or within, the indent. The other (or second) end, may be spaced from the indent and positioned more towards the periphery of the sole portion. Panels on the sole portion of the sock, such as the first, second, third and/or fourth panels (as described in any form above) may cross the indent or converge at the indent.

In a preferred embodiment, the support has a lower leg section that, in use, conforms to at least a portion of the wearer's lower leg. Preferably, in use, the support extends to the knee, or immediately below the knee of the wearer, most preferably not extending above the knee. The support may thus have a top edge defining a top opening through which the wearer inserts their foot or leg when donning the support. The lower leg section may comprise one or more fastenings that facilitate donning of the support, for example one or more zips.

The support may substantially cover the shin and the calf muscle of the wearer. However, the sock may have an opening defined through a posterior or back leg portion of the sock. An example of such an opening is described in international patent application WO2010/139939A1. The posterior opening may thus expose a substantial portion of the wearer's calf muscle, particularly the lower portion of the calf muscle, when the sock is worn. The opening may be substantially ovoid or elliptical.

In a particularly preferred embodiment, the support, when worn, urges dorsiflexion of the wearer's foot, such that the wearer's toes are urged towards the shin, and the wearer's calf muscle is stretched. Stretching the wearer's calf muscle may be particularly beneficial for the management of plantar fasciitis.

Preferably, the support comprises a resilient reinforcement panel or group of panels that assists dorsiflexion of the wearer's foot. The panel or panels, which may be termed a dorsiflexion panel or panels, may urge the foot upwards towards the shin, pivoting at the ankle joint. A dorsiflexion panel may extend down a front part of the lower leg section of the sock, such that, in use, it extends down the wearer's shin. Preferably, the dorsiflexion panel also extends along at least part of a top-of-foot section of the sock, such that, in use, it extends along at least part of the foot, preferably the top or upper side, of the foot. The dorsiflexion panel may extend to the toe section.

The dorsiflexion panel may extend around the circumference of the sock at the upper-most part of the lower leg section, immediately below the top opening, or top edge. This may allow the device to grip the leg of the wearer by, for example, constricting the device around the top of the wearer's calf muscle.

Preferably, part of the back of the lower leg section, or the calf section, of the sock does not substantially have a resilient panel positioned over it. So, in use, there may not be a resilient panel covering the lower part or the belly of the wearer's calf muscle. The presence of a dorsiflexion panel on the front of the lower-leg section and the absence of a panel on the back of the lower-leg section may assist in dorsiflexion of the wearer's foot, in use.

The device may have a dorsiflexion dart or join positioned at the front of the support, where, for example, the lower leg section of the sock joins the foot section of the sock. This may be positioned, in use, at the front of the wearer's ankle. The resulting seam, or dorsiflexion seam, may be positioned at the apex of the angle created between the front of the lower leg section and the top of the foot section. The dart or join, in use, may thus encourage dorsiflexion of the wearer's foot, in particular when it may be present in conjunction with the dorsiflexion panel. The dorsiflexion dart or join may be manufactured by, for example, folding or pleating a part of the front of the sock and joining edges of the fold, or cutting out a section of the front of the sock to create a hole or opening and then joining the edges of the opening together, for example by stitching. Preferably, the dorsiflexion panel is joined to the sock, prior to creating the dorsiflexion dart or seam.

In some embodiments the support may be a bespoke support designed to support the management of plantar fasciitis, which may assist in reducing the symptoms of plantar fasciitis. The position and strength of reinforcements may be specific for an individual wearer. Thus, a clinician may specify properties of a support.

Preferably, the support allows the full range of active foot and ankle movement, even though forces may act to urge the foot, or parts of the foot, in a specific direction.

Advantageously, the support may not comprise fasteners for securing the support to the wearers leg, such as Velcro® straps, zips or buttons. This may allow quicker application of the support.

The invention provides a method of making an orthotic support for the management or treatment of plantar fasciitis comprising the steps of forming a sock from resilient material for conforming to a wearer's foot, and forming a dart in a sole portion of the sock such that a portion of the sole portion is indented relative to the remainder of the sole portion of the sock.

A dart may be formed by pleating or gathering material at the sole portion to form an indent in the sole portion. A dart may be formed by removing a portion of material from a sole portion of the sock to leave a hole defined through the sole portion of the sock, and joining edges of the hole such that a portion of the sole portion is indented relative to the remainder of the sock.

The method may thus form an indent or concavity in the sock, as described in any form above.

Where a dart is formed by removing material to form a hole and joining edges of the hole, the hole is preferably an oval shape, with the longitudinal axis of the oval extending along a longitudinal axis of the foot.

Preferably the longest dimension of the hole is between 3cm and 20cm in length, for example between 5cm and 15cm, or between 7cm and 10cm.

Preferably the shortest dimension of the hole is between 1cm and 6cm, for example between 2cm and 5cm.

The method may include the step of assessing a patient, determining the required closure force that a support needs to apply to their foot to help alleviate plantar fasciitis, and specifying the required dimensions of a hole to form an indent giving the desired properties.

Preferably, reinforcement panels are attached to the sole portion of the sock prior to the step of joining edges of the hole.

The method may comprise attaching, joining or sewing reinforcement panels to the sock, the reinforcement panels being positioned as described in any form above. For example, the method may also comprise attaching a dorsiflexion panel to a front part of a lower-leg section of the sock.

The invention may provide a method of managing plantar fasciitis. This may involve treating, preventing or ameliorating plantar fasciitis or the symptoms thereof. The method may comprise wearing an orthotic support as described in any form above. Preferably, the orthotic device is worn for a period of between 10 and 20 hours per day. Preferably, the orthotic device is not worn overnight, or when a patient is asleep.

### Specific Description of a Preferred Embodiment

An embodiment of the invention will now be described, by way of example, and with reference to the accompanying drawings, in which:
Figure 1 shows a side projection of a support according to an embodiment of the invention, in a flat, unworn configuration;
Figure 2 shows a bottom projection, or lower plan view, of the support of Figure 1;
Figure 3 shows a front projection of a support according to another embodiment of the invention;
Figure 4 shows a rear projection of the support of Figure 3;
Figure 5 shows a right side projection of the support of Figures 3 and 4, in a flat, unworn configuration;
Figure 6 shows a left side projection of the support of Figures 3 to 5, in a flat, unworn configuration;
Figure 7 shows a bottom projection, or lower plan view, of the support of Figures 3 to 6.

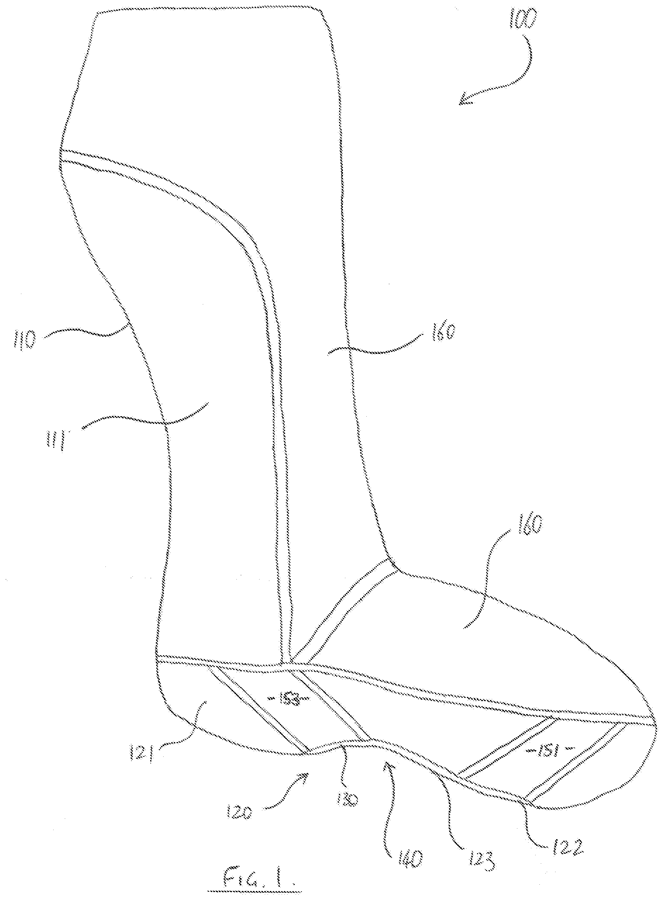

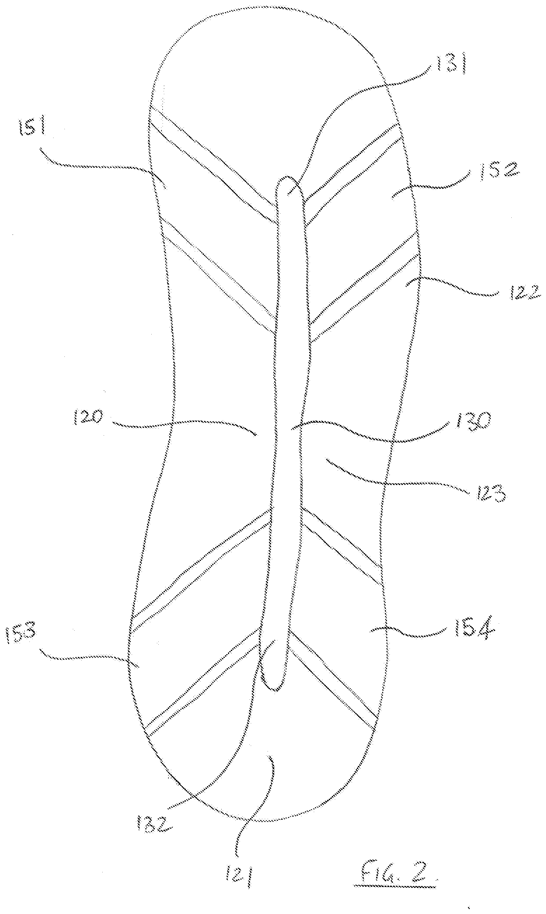

A dynamic orthotic support according to a specific embodiment of the invention is illustrated in Figures 1 and 2. The orthotic support 100 comprises a sock 110 formed from an elastomeric underlying material 111. The sock 110 has a sole portion 120 (as seen, in particular, in lower plan view in Figure 2). The sole portion 120 may be said to comprise a heel section 121, which is the section of the sole portion contacting the heel of a wearer, a ball-of-foot section 122, which is the section contacting the ball of a wearer's foot and base of a wearer's toes, and an arch section 123, which is the section contacting a wearer's arch.

The sole portion 120 is shaped by means of a dart or indent seam 130 to produce an indent 140 in the arch section of the sole portion. Four sole reinforcement panels 151, 152, 153, 154 are attached to the sock 110 and traverse the sole portion 120 in substantially diagonal configuration. First 151 and second 152 sole reinforcement panels extend from right and left sides of the ball of foot section of the sole portion diagonally rearward to a toe-end 131 of the indent seam 130. Third 153 and fourth 154 sole reinforcement panels extend from right and left sides of the heel section of the sole portion diagonally towards to a heel-end 132 of the indent seam 130.

A dorsiflexion assist panel 160 is attached to a front of shin and upper foot portion of the support. The dorsiflexion panel and the sole reinforcement panels are formed from a resilient elastomeric material that is stitched to the underlying material.

The indent seam or dart 130 was formed by removing a substantially oval shaped section of material from the sole portion 120 and then rejoining the edges of the hole formed by removal of the material. The result of this procedure is to form an indent 140 into the sole portion 120 of the support. The four sole reinforcement panels 151, 152, 153, 154 extend into the indent.

When the support is worn, the material at the indent stretches to a greater degree in order to conform to a wearer's foot than material not forming part of the indent. This initiates lines of tension in the sole portion which, due to the shape of the indent, act to urge the wearer's foot towards a closed position.

The lines of tension initiated by the indent are augmented by the four sole reinforcement panels which act to urge the wearer's foot closed, both laterally and transversely. The dynamic action that urges the foot closed may help alleviate symptoms of plantar fasciitis. The dorsiflexion assist panel may further alleviate symptoms of plantar fasciitis by promoting dorsiflexion of the wearer's foot, and stretching of the wearer's calf muscle.

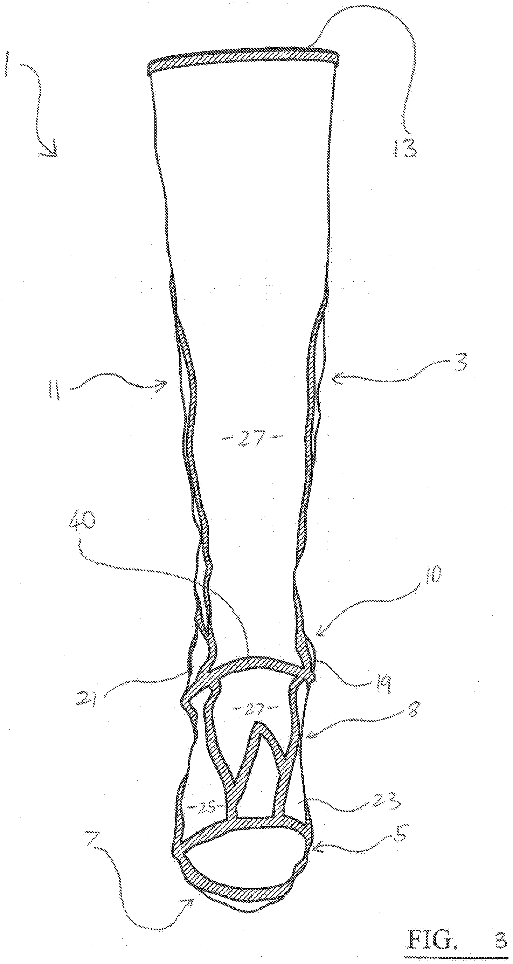

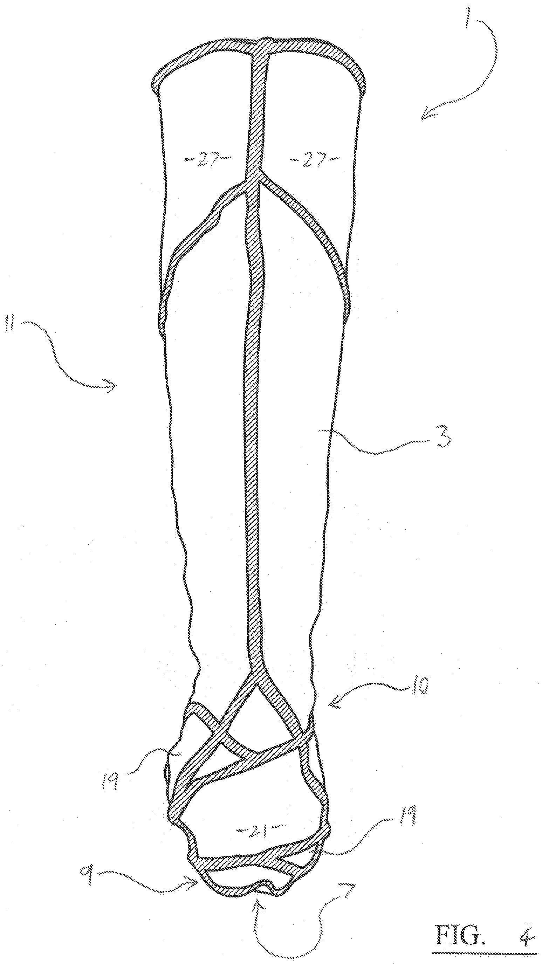

A further specific embodiment of a dynamic orthotic support is illustrated in Figures 3 to 7.

A support 1, as shown in Figures 3-7, comprises a sock 3 manufactured from a resilient elastomeric material having a composition of 51% polyamide, 17% cotton and 32% Dorlastan®. The material is a lightweight breathable elastomeric fabric and is suitable for forming the underlying material of the support. Other suitable fabrics are available, for example under the trade names of Spandex® or Lycra®.

The sock has a sole portion 7. The sock may further be said to define a toe section 5, an upper foot section 8, a heel section 9, an ankle section 10 and a lower leg section 11. The top of the lower leg section defines an opening 13 to an inside of the sock.

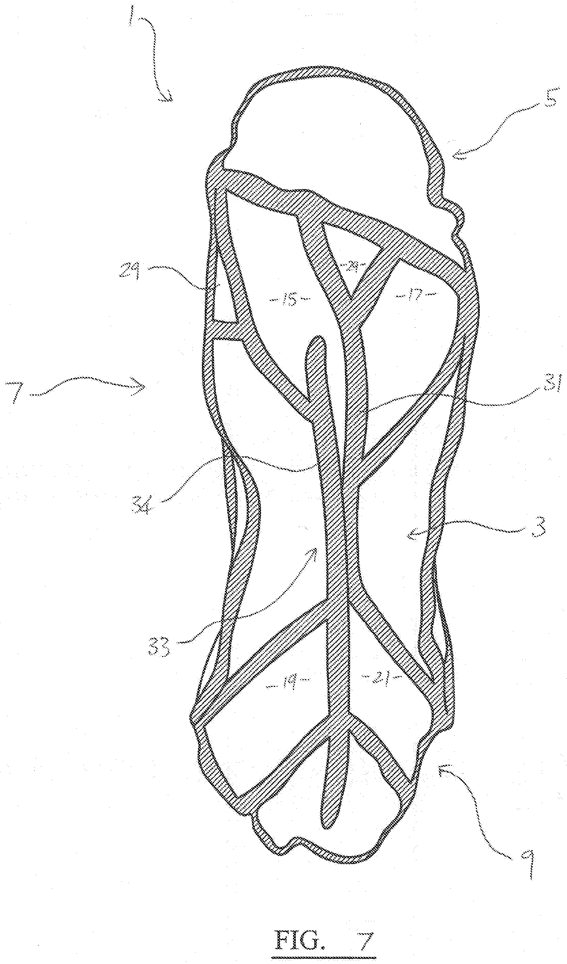

On the sole portion 7 of the sock 3 (which is shown most clearly in Figure 7), a first panel, or first sole reinforcement panel 15, extends from a right side of the toe section 5 to a central seam 31 that runs substantially longitudinally and centrally along the sole portion. A second panel, or second sole reinforcement panel, 17 on the sole portion of the sock extends from the left side of the toe section to the central seam. The first and second panels form a V-shape on the sole portion, with the apex of this "V" pointing rearward towards, heel section of the sock.

A third panel, or third sole reinforcement panel, 19 on the sole portion 7 of the sock extends from the central seam 31 backwards and rightwards to a right side of the heel section 9 on the sole portion. The third panel also extends upwardly and across the back of the heel section, around to the left side of the heel section and to the left side of the ankle section 10. As it crosses the heel section, the panel widens so that it extends over the heel section and ankle section.

A fourth panel, or fourth sole reinforcement panel, 21 on the sole portion 7 of the sock extends from the central seam 31 backwards and leftwards to a left side of the heel section 9 on the sole portion. The fourth panel also extends upwardly and across the back of the heel section, around to the right side of the heel section and to the right side of the ankle section. As it crosses the heel section, the panel widens so that it extends over the heel section and ankle section.

On the sole portion 7, the third and fourth panels form a V-shape with an apex that points forwards, towards the toe section. The apex formed by the third and fourth panels is nearer to the heel section, i.e. further back, than the apex formed by the first and second panels. The apex formed by the third and fourth panels does not meet or contact the apex formed by the first and second panels.

A fifth panel 23 or left side panel, extends from a left side of the heel section to the toe section, along a left side of the upper foot section 8 of the sock. Similarly, a sixth panel 25 or right side panel, extends from a right side of the heel section 9 to the toe section 5, along a right side of the upper foot section of the sock.

The fifth and sixth panels 23, 25 may be continuous with or attached to the third and fourth panels respectively.

A dorsiflexion panel 27 extends from the opening 13 of the sock, down the front of the lower leg section 11, to the upper foot section 8. On the upper foot section, the dorsiflexion panel splits into a V-shape and extends to the right and left side of the toe section. At the top of the sock, the dorsiflexion panel extends around the circumference of the sock. Further down the lower leg section of the sock, the dorsiflexion panel only extends down to the front (or shin section), and does not cover the back (or calf section).

The support has a dorsiflexion seam 40, extending around the front of the support, positioned at the apex of the angle defined between the front of the lower leg section 11 and the upper foot section 8. The dorsiflexion seam is a dorsiflexion dart or join, formed by removing a section material at the front of the support to create a hole and joining edges of the hole together. The use of a dart increases the dorsiflexion force applied to a wearer's foot.

A cross-panel 29 extends across the sole portion 7 of the sock, underneath the first and the second panels, behind the toe section.

All of the reinforcement panels 15, 17, 19, 21, 23, 25 are fabricated from a resilient material having a composition of 81% polyamide and 19% Lycra® with an elastomeric material that offers a greater resistance to deformation than the underlying elastomeric material of the sock. The panels are attached to the sock, or underlying panels, by stitching their edges to the sock or to underlying panels. The edges of the panels are defined by seams.

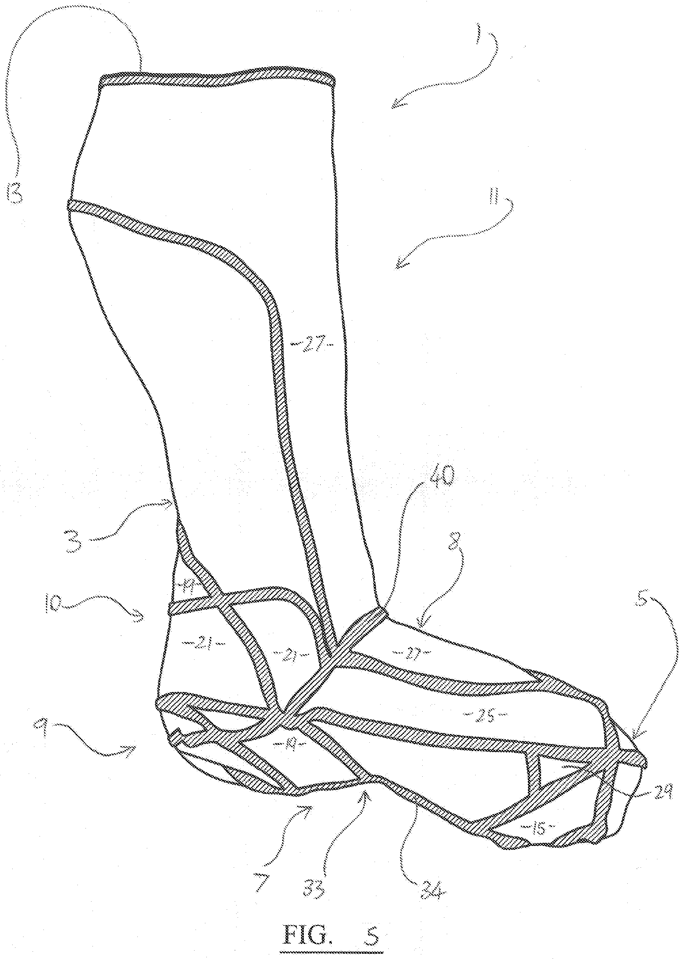

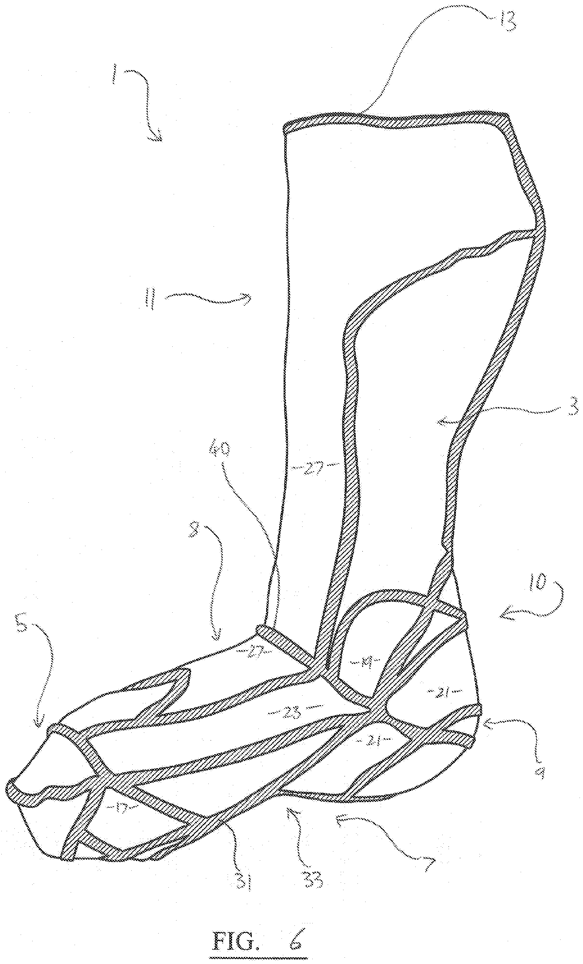

The sole portion 7 of the sock 3 has a roughly conical indent 33 that extends substantially centrally into the sole portion, between the heel section 9 and the toe section 5. This is shown most clearly in Figures 5 and 6. The indent is formed at an indent seam or indent dart 34. The indent seam is slightly offset with respect to the central seam 31 in this specific example. The first 15, second 17, third 19 and fourth 21 panels converge at the indent such that the apex formed by the first and second panels and the apex formed by the third and fourth panels are positioned at, or within, the indent. The indent is formed by removing a section of the material from the support to create a hole, and joining the edges of the hole together to form the indent dart or indent seam.

In use, a wearer inserts their foot and lower leg through the opening 13 at the top of the sock, such that the sock covers and conforms to the wearer's foot and lower leg. The foot and lower leg are appropriately placed in the sock such that the toe section 5 conforms to and covers the wearer's toes, the upper foot section 8 covers and conforms to the wearer's upper foot, the sole portion 7 conforms to the wearer's sole or plantar aspect, the heel section conforms to and covers the wearer's heel or calcaneus, the ankle section 10 conforms to and covers a wearer's ankle, and a lower leg section 11 covers and conforms to the wearer's lower leg, including the shin, calf muscle and Achilles tendon. The edge of the sock defining the opening 13 sits just below the wearer's knee.

When the wearer's leg and foot is placed into the sock, the first panel 15 extends from the ball of the foot on a right side of the wearer's sole to a substantially central position (with respect to the width, or lateral axis, of the foot), further back on the sole. Similarly, the second panel 17 extends from the ball of the foot, on a left side sole to a substantially central position further back on the sole.

The third panel 19, extends from the wearer's heel on the right side of the sole, to a substantially central position further forwards on the sole and the fourth panel 21 extends from the wearer's heel on the left side of the sole, to a substantially central position, further forwards on the sole. The central position on the sole to which each of the panels 15, 17, 19, 21 is at or within the longitudinal arch of the foot, between the ball of the foot and the heel. The indent 33 is positioned at, or along the wearer's longitudinal arch.

The third panel 19 wraps around the back of the wearer's heel to a left side of the heel. Widening of the third panel means that it also covers at least part of the wearer's left ankle. The fourth panel 21 also wraps around the back of the wearer's heel to a right side of the heel. Widening of the fourth panel means that it covers at least part of the wearer's right ankle.

The fifth and sixth panels 23, 25 extend from the wearer's heel along a right side and a left side of the top of the wearer's foot, respectively, up to the base of the toes.

The cross panel 29 extends across the ball of the wearer's foot.

The dorsiflexion panel 27 extends down the wearer's shin from just below the knee to the base of the toes at the top of the wearer's foot. The dorsiflexion panel at the top part of the sock, immediately underneath the opening 13 of the sock, surrounds the leg such that it covers the upper shin and upper calf of the wearer. On the lower part of the wearer's lower leg, the dorsiflexion panel only covers the shin.

The panels 15, 17, 19, 21, 23, 25 and indent 33 exert directional forces that urge the wearer's foot into a specific configuration or orientation. The support urges the wearer's foot to close, shortening the distance between the ball of the foot and the heel and increasing the height of the longitudinal arch of the foot and increasing the height of the transverse arch of the foot. This results in an off-loading of the plantar fascia, decreasing the strain on the plantar fascia.

The support also causes dorsiflexion by urging the wearer's toes towards the shin, resulting from the action of the dorsiflexion panel 27 and the dorsiflexion seam 40. This allows stretching of the wearer's calf muscle.

## Claims

1. A dynamic orthotic support (1,100) for the management of plantar fasciitis, comprising a sock (3,110) having a sole portion (7,120) formed from a resilient material (111), in which the sole portion (7,120) is shaped such that, when worn, lines of tension are initiated in the sole portion (7,120) that act to urge a wearer's foot towards a closed position, for alleviating the symptoms of plantar fasciitis,
**characterised in that** the sole portion (7,120) comprises a dart (34,130) formed from gathering or pleating material at the sole portion (7,120) and joining the gathered or pleated material, or by removing a portion of material from the sole portion (7,120) thereby forming a hole defined through the sole portion (7,120), and joining edges of the hole, the dart (34,130) forming an indent (33, 140) in the sole portion (7, 120).

2. An orthotic support according to claim 1, in which the sole portion (7, 120) comprises a heel section (9, 121), a ball-of-foot section (122), and an arch section (123); the sole portion (7, 120) being shaped such that, when worn, material of the arch section is stretched by a greater proportion than material of the heel section (9, 121) or material of the ball-of-foot section (122).

3. An orthotic support according to claim 1 or claim 2, in which the indent (33, 140) is a substantially conical indent that, when stretched to conform the wearer's foot, initiates lines of tension that act to close the wearer's foot.

4. An orthotic support according to claim 2 or claim 3, in which the dart (34, 130) runs substantially centrally along the sole portion (7, 120), optionally in which the dart (34, 130) extends between the ball-of-foot section (122) of the sole portion (7, 120) and to the heel section (9, 121) of the sole portion.

5. An orthotic support according to any preceding claim comprising at least one resilient reinforcement panel (15, 17, 19, 21, 151, 152, 153, 154) attached to the sole portion (7, 120) of the sock in order to, when the support is worn, urge a wearer's foot towards a closed position.

6. An orthotic support according to claim 5, in which the, or each, resilient reinforcement panel (15, 17, 19, 21, 151, 152, 153, 154), when the support is worn, exerts a contractile force or line of tension diagonally across at least part of the sole portion (7, 120).

7. An orthotic support according to claim 5 or claim 6, in which:
a) a resilient reinforcement panel (15, 151) extends diagonally rearwards across at least part of the sole portion (7, 120) from a right side of a ball-of-foot section (122) of the sole portion (7, 120), optionally, in which the resilient reinforcement panel (15, 151) extends from the right side of the ball-of-foot section (122) to a position on the sole portion (7, 120) that is substantially equidistant from a right side and a left side of the sole portion (7, 120);
b) a resilient reinforcement panel (17, 152) extends diagonally rearwards across at least part of the sole portion (7, 120) from a left side of a ball-of-foot section (122) of the sole portion (7, 120), optionally in which the resilient reinforcement panel (17, 152) extends from the left side of the ball-of-foot section (122) to a position on the sole portion (7, 120) that is substantially equidistant from a right side and a left side of the sole portion (7, 120);
c) a resilient reinforcement panel (21, 154) extends diagonally forwards across at least part of the sole portion (7, 120) of the sock (3, 110) from a left side of a heel section (9, 121) of the sole portion (7, 120), optionally in which the resilient reinforcement panel (21, 154) extends from the left side of the heel section (9, 121) of the sole portion (7, 120) of the sock (3, 110) to a position on the sole portion (7, 120) that is substantially equidistant from a right side and a left side of the sole portion (7, 120); and/or
d) a resilient reinforcement panel (19, 153) extends diagonally forwards across at least part of the sole portion (7, 120) from a right side of a heel section (9, 121) of the sole portion (7, 120), optionally in which the resilient reinforcement panel (19, 153) extends from the right side of the heel section (9, 121) of the sole portion (7, 120) to a position on the sole portion (7, 120) that is substantially equidistant from a right side and a left side of the sole portion (7, 120).

8. An orthotic support according to any of claims 5 to 7, in which the, or each, resilient reinforcement panel (15, 17, 19, 21, 151, 152, 153, 154) on the sole portion (7, 120) has a part that extends to an arch section (123) on the sole portion (7, 120), preferably to the indent (33, 140) in the sole portion (7, 120).

9. An orthotic support according to any preceding claim comprising at least one resilient reinforcement panel (23, 25) extending across a heel portion (9) of the sock (3, 110), and/or comprising at least one resilient reinforcement panel (27) attached to the sock such that, when worn, dorsiflexion of a wearer's foot is promoted.

10. A method of making an orthotic support (1, 100) for the management of plantar fasciitis, comprising the steps of forming a sock (3, 110) from resilient material (111) for conforming to a wearer's foot, **characterised in that** the method comprises forming a dart (34, 130) in a sole portion (7, 120) of the sock (3, 110) such that a portion of the sole portion (7, 120) is indented relative to the remainder of the sock (3, 110), wherein the dart (34, 130) is formed by gathering or pleating material at the sole portion (7, 120) of the sock (3, 110) and joining the gathered or pleated material to form the dart (34, 130), or by removing a portion of material from the sole portion (7, 120) thereby forming a hole defined through the sole portion, and joining edges of the hole to form the dart (34, 130).

11. A method according to claim 10, comprising the step of attaching at least one resilient reinforcement panel (15, 17, 19, 21, 151, 152, 153, 154) to the sole portion (7, 120) of the sock (3, 110), preferably prior to the step of forming the dart (34, 130), optionally, in which the, or each, resilient reinforcement panel (15, 17, 19, 21, 151, 152, 153, 154) is attached to the sole portion (7, 120) of the sock (3, 110) in a diagonal orientation in order to exert a contractile force or line of tension diagonally across and along at least part of the sole portion (7, 120) of the sock (3, 110) when the support is worn.

12. A method according to claim 11, in which:
a) a resilient reinforcement panel (15, 151) attached to the sock (3, 110) extends diagonally rearward across at least part of the sole portion (7, 120) from a right side of a ball-of-foot section (122) of the sole portion (7, 120), optionally in which the resilient reinforcement panel (15, 151) is attached such that it extends from the right side of the ball-of-foot section (122) to a position on the sole portion (7, 120) that is substantially equidistant from a right side and a left side of the sole portion (7, 120);
b) a resilient reinforcement panel (17, 152) attached to the sock (3, 110) extends diagonally rearward across at least part of the sole portion (7, 120) from a left side of a ball-of-foot section (122) of the sole portion (7, 120), optionally in which the resilient reinforcement panel (17, 152) is attached such that it extends from the left side of the ball-of-foot section (122) to a position on the sole portion (7, 120) that is substantially equidistant from a right side and a left side of the sole portion (7, 120);
c) a resilient reinforcement panel (21, 154) attached to the sock (3, 110) extends diagonally forward across at least part of the sole portion (7, 120) of the sock (3, 110) from a left side of a heel section (9, 121) of the sole portion (7, 120), optionally in which the resilient reinforcement panel (21, 154) is attached such that it extends from the left side of the heel section (9, 121) of the sole portion (7, 120) of the sock (3, 110) to a position on the sole portion (7, 120) that is substantially equidistant from a right side and a left side of the sole portion (7, 120); and/or
d) a resilient reinforcement panel (19, 153) attached to the sock (3, 110) extends diagonally forward across at least part of the sole portion (7, 120) from a right side of a heel section (9, 121) of the sole portion (7, 121), optionally in which the resilient reinforcement panel (19, 153) is attached such that it extends from the right side of the heel section (9, 121) of the sole portion (7, 120) to a position on the sole portion (7, 120) that is substantially equidistant from a right side and a left side of the sole portion (7, 120).

13. A method according to claim 11, in which the, or each, resilient reinforcement panel (15, 17, 19, 21, 151, 152, 153, 154) is attached to the sole portion (7, 120) of the sock (3, 110) so as to have a part, or end, that extends to an arch section (123) on the sole portion (7, 120), preferably to the dart (34, 130) in the sole portion (7, 120).

14. A method according to any of claims 11 to 13 comprising the steps of attaching at least one resilient reinforcement panel (23, 25) across a heel portion (9, 121)) at a back of the sock (3, 110), and/or attaching a resilient dorsiflexion-assist reinforcement panel (27) that, when worn, promotes dorsiflexion of a wearer's foot, optionally in which the dorsiflexion-assist reinforcement panel (27) is attached to the sock (3, 110) such that it extends from a shin section of the sock to a top-of-foot section of the sock (3, 110).

15. A method according to claim 10 and any preceding claim dependent on claim 10, in which the hole is substantially oval in shape with the longest axis of the oval running in a heel-to-toe direction, optionally in which the longest dimension of the hole is between 3cm and 20cm, and the shortest dimension of the hole is between 1cm and 6cm.

## Patentansprüche

1. Dynamische orthetische Stütze (1, 100) zur Behandlung von Plantarfasziitis, die einen Strumpf (3, 110) aufweist, der einen aus einem elastischen Material (111) gebildeten Sohlenteil (7, 120) hat, bei dem der Sohlenteil (7, 120) so gestaltet ist, dass beim Tragen Zugspannungslinien im Sohlenteil (7, 120) veranlasst werden, die wirken, um den Fuß eines Trägers auf eine geschlossene Stellung hin zu drängen, um die Symptome von Plantarfasziitis abzubauen,
**dadurch gekennzeichnet, dass** der Sohlenteil (7, 120) einen durch Raffen oder Fälteln von Material am Sohlenteil (7, 120) und Zusammenfügen des gerafften oder gefältelten Materials oder durch Entfernen eines Materialteils aus dem Sohlenteil (7, 120), um dadurch ein durch den Sohlenteil (7, 120) definiertes Loch zu bilden, und Zusammenfügen von Rändern des Lochs gebildeten Abnäher (34, 130) aufweist, wobei der Abnäher (34, 130) im Sohlenteil (7, 120) einen Einzug (33, 140) bildet.

2. Orthetische Stütze nach Anspruch 1, bei der der Sohlenteil (7, 120) einen Fersenabschnitt (9, 121), einen Fußballenabschnitt (122) und einen Fußgewölbeabschnitt (123) aufweist; wobei der Sohlenteil (7, 120) so gestaltet ist, dass beim Tragen Material des Fußgewölbeabschnitts um einen größeren Anteil gedehnt wird als Material des Fersenabschnitts (9, 121) oder Material des Fußballenabschnitts (122).

3. Orthetische Stütze nach Anspruch 1 oder Anspruch 2, bei der der Einzug (33, 140) ein im Wesentlichen kegelförmiger Einzug ist, der, wenn er gedehnt wird, um sich an den Fuß des Trägers anzupassen, Zugspannungslinien veranlasst, die zum Schließen des Fußes des Trägers wirken.

4. Orthetische Stütze nach Anspruch 2 oder Anspruch 3, bei der der Abnäher (34, 130) im Wesentlichen mittig am Sohlenteil (7, 120) entlang verläuft, wahlweise bei der der Abnäher (34, 130) sich zwischen dem Fußballenabschnitt (122) des Sohlenteils (7, 120) und dem Fersenabschnitt (9, 121) des Sohlenteils erstreckt.

5. Orthetische Stütze nach einem der vorhergehenden Ansprüche, die wenigstens ein elastisches Verstärkungsstück (15, 17, 19, 21, 151, 152, 153, 154) aufweist, das am Sohlenteil (7, 120) des Strumpfs angebracht ist, um beim Tragen der Stütze den Fuß eines Trägers zu einer geschlossenen Stellung hin zu drängen.

6. Orthetische Stütze nach Anspruch 5, bei der das oder jedes elastische Verstärkungsstück (15, 17, 19, 21, 151, 152, 153, 154) beim Tragen der Stütze eine zusammenziehende Kraft oder Zugspannungslinie diagonal über wenigstens einen Teil des Sohlenteils (7, 120) ausübt.

7. Orthetische Stütze nach Anspruch 5 oder Anspruch 6, bei der:
a) ein elastisches Verstärkungsstück (15, 151) sich von einer rechten Seite eines Fußballenabschnitts (122) des Sohlenteils (7, 120) diagonal nach hinten über wenigstens einen Teil des Sohlenteils (7, 120) erstreckt, wahlweise bei der sich das elastische Verstärkungsstück (15, 151) von der rechten Seite des Fußballenabschnitts (122) zu einer Position am Sohlenteil (7, 120) erstreckt, die zu einer rechten Seite und einer linken Seite des Sohlenteils (7, 120) im Wesentlichen abstandsgleich ist;
b) ein elastisches Verstärkungsstück (17, 152) sich von einer linken Seite eines Fußballenabschnitts (122) des Sohlenteils (7, 120) diagonal nach hinten über wenigstens einen Teil des Sohlenteils (7, 120) erstreckt, wahlweise bei der sich das elastische Verstärkungsstück (17, 152) von der linken Seite des Fußballenabschnitts (122) zu einer Position am Sohlenteil (7, 120) erstreckt, die zu einer rechten Seite und einer linken Seite des Sohlenteils (7, 120) im Wesentlichen abstandsgleich ist;
c) ein elastisches Verstärkungsstück (21, 154) sich von einer linken Seite eines Fersenabschnitts (9, 121) des Sohlenteils (7, 120) diagonal nach vorn über wenigstens einen Teil des Sohlenteils (7, 120) des Strumpfs (3, 110) erstreckt, wahlweise bei der das elastische Verstärkungsstück (21,154) sich von der linken Seite des Fersenabschnitts (9, 121) des Sohlenteils (7, 120) des Strumpfs (3,110) zu einer Position am Sohlenteil (7, 120) erstreckt, die zu einer rechten Seite und einer linken Seite des Sohlenteils (7, 120) im Wesentlichen abstandsgleich ist; und/oder
d) ein elastisches Verstärkungsstück (19, 153) sich von einer rechten Seite eines Fersenabschnitts (9, 121) des Sohlenteils (7, 120) diagonal nach vorn über wenigstens einen Teil des Sohlenteils (7, 120) erstreckt, wahlweise bei der das elastische Verstärkungsstück (19, 153) sich von der rechten Seite des Fersenabschnitts (9, 121) des Sohlenteils (7, 120) zu einer Position am Sohlenteil (7, 120) erstreckt, die zu einer rechten Seite und einer linken Seite des Sohlenteils (7, 120) im Wesentlichen abstandsgleich ist.

8. Orthetische Stütze nach einem der Ansprüche 5 bis 7, bei der das oder jedes elastische Verstärkungsstück (15, 17, 19, 21, 151, 152, 153, 154) am Sohlenteil (7, 120) einen Teil hat, der sich zu einem Gewölbeabschnitt (123) am Sohlenteil (7, 120), vorzugsweise zum Einzug (33, 140) im Sohlenteil (7, 120) erstreckt.

9. Orthetische Stütze nach einem der vorhergehenden Ansprüche, die wenigstens ein elastisches Verstärkungsstück (23, 25) aufweist, das sich über einen Fersenteil (9) des Strumpfs (3, 110) erstreckt; und/oder wenigstens ein elastisches Verstärkungsstück (27) aufweist, das so an dem Strumpf angebracht ist, dass beim Tragen die Dorsalflexion des Fußes eines Trägers gefördert wird.

10. Verfahren zur Anfertigung einer orthetischen Stütze (1, 100) zur Behandlung von Plantarfasziitis, das die Schritte des Herstellens eines Strumpfs (3, 110) aus elastischem Material (111) zum Anpassen an den Fuß eines Trägers aufweist, **dadurch gekennzeichnet, dass** das Verfahren das Herstellen eines Abnähers (34, 130) in einem Sohlenteil (7, 120) des Strumpfs (3, 110) aufweist, so dass ein Teil des Sohlenteils (7, 120) relativ zum übrigen Strumpf (3, 110) eingezogen wird, wobei der Abnäher durch Raffen oder Fälteln von Material am Sohlenteil (7, 120) des Strumpfs (3, 110) und Zusammenfügen des gerafften oder gefältelten Materials zum Herstellen des Abnähers (34, 130) oder durch Entfernen eines Materialteils aus dem Sohlenteil (7, 120), um dadurch ein durch den Sohlenteil definiertes Loch zu bilden, und Zusammenfügen von Rändern des Lochs zum Herstellen des Abnähers (34, 130) hergestellt wird.

11. Verfahren nach Anspruch 10, das den Schritt des Anbringens von wenigstens einem elastischen Verstärkungsstück (15, 17, 19, 21, 151, 152, 153, 154) am Sohlenteil (7, 120) des Strumpfs (3, 110), vorzugsweise vor dem Schritt des Herstellens des Abnähers (34, 130), aufweist, wahlweise bei dem das oder jedes elastische Verstärkungsstück (15, 17, 19, 21, 151, 152, 153, 154) in einer diagonalen Ausrichtung am Sohlenteil (7, 120) des Strumpfs (3, 110) angebracht wird, um beim Tragen der Stütze eine zusammenziehende Kraft oder Zugspannungslinie diagonal über und auf wenigstens einen Teil des Sohlenteils (7, 120) des Strumpfs (3, 110) auszuüben.

12. Verfahren nach Anspruch 11, bei dem:
a) ein an dem Strumpf (3, 110) angebrachtes elastisches Verstärkungsstück (15, 151) sich von einer rechten Seite eines Fußballenabschnitts (122) des Sohlenteils (7, 120) diagonal nach hinten über wenigstens einen Teil des Sohlenteils (7, 120) erstreckt, wahlweise bei dem das elastische Verstärkungsstück (15, 151) so angebracht wird, dass es sich von der rechten Seite des Fußballenabschnitts (122) zu einer Position am Sohlenteil (7, 120) erstreckt, die zu einer rechten Seite und einer linken Seite des Sohlenteils (7, 120) im Wesentlichen abstandsgleich ist;
b) ein an dem Strumpf (3, 110) angebrachtes elastisches Verstärkungsstück (17, 152) sich von einer linken Seite eines Fußballenabschnitts (122) des Sohlenteils (7, 120) diagonal nach hinten über wenigstens einen Teil des Sohlenteils (7, 120) erstreckt, wahlweise bei dem das elastische Verstärkungsstück (17, 152) so angebracht wird, dass es sich von der linken Seite des Fußballenabschnitts (122) zu einer Position am Sohlenteil (7, 120) erstreckt, die zu einer rechten Seite und einer linken Seite des Sohlenteils (7, 120) im Wesentlichen abstandsgleich ist;
c) ein an dem Strumpf (3, 110) angebrachtes elastisches Verstärkungsstück (21, 154) sich von einer linken Seite eines Fersenabschnitts (9, 121) des Sohlenteils (7, 120) diagonal nach vorn über wenigstens einen Teil des Sohlenteils (7, 120) des Strumpfs (3, 110) erstreckt, wahlweise bei dem das elastische Verstärkungsstück (21,154) so angebracht wird, dass es sich von der linken Seite des Fersenabschnitts (9, 121) des Sohlenteils (7, 120) des Strumpfs (3,110) zu einer Position am Sohlenteil (7, 120) erstreckt, die zu einer rechten Seite und einer linken Seite des Sohlenteils (7, 120) im Wesentlichen abstandsgleich ist; und/oder
d) ein an dem Strumpf (3, 110) angebrachtes elastisches Verstärkungsstück (19, 153) sich von einer rechten Seite eines Fersenabschnitts (9, 121) des Sohlenteils (7, 120) diagonal nach vorn über wenigstens einen Teil des Sohlenteils (7, 120) erstreckt, wahlweise bei dem das elastische Verstärkungsstück (19, 153) so angebracht wird, dass es sich von der rechten Seite des Fersenabschnitts (9, 121) des Sohlenteils (7, 120) zu einer Position am Sohlenteil (7, 120) erstreckt, die zu einer rechten Seite und einer linken Seite des Sohlenteils (7, 120) im Wesentlichen abstandsgleich ist.

13. Verfahren nach Anspruch 11, bei dem das oder jedes elastische Verstärkungsstück (15, 17, 19, 21, 151, 152, 153, 154) am Sohlenteil (7, 120) des Strumpfs (3, 110) angebracht wird, um einen Teil oder ein Ende zu haben, der bzw. das sich zu einem Gewölbeabschnitt (123) am Sohlenteil (7, 120), vorzugsweise zum Abnäher (34, 130) im Sohlenteil (7, 120) erstreckt.

14. Verfahren nach einem der Ansprüche 11 bis 13, das die Schritte des Anbringens von wenigstens einem elastischen Verstärkungsstück (23, 25) über einen Fersenteil (9, 121) hinten an dem Strumpf (3, 110) und/oder des Anbringens eines elastischen Dorsalflexionshilfe-Verstärkungsstücks (27) aufweist, das beim Tragen die Dorsalflexion des Fußes eines Trägers fördert, bei dem wahlweise das Dorsalflexionshilfe-Verstärkungsstück (27) so an dem Strumpf (3, 110) angebracht wird, dass es sich von einem Schienbeinabschnitt des Strumpfs bis zu einem Fußrückenabschnitt des Strumpfs (3, 110) erstreckt.

15. Verfahren nach Anspruch 10 und einem der vorhergehenden von Anspruch 10 abhängigen Ansprüche, bei dem das Loch im Wesentlichen eine ovale Form hat, wobei die längste Achse des Ovals in einer Richtung von Ferse zu Zehen verläuft, wahlweise bei dem die längste Abmessung des Lochs zwischen 3 cm und 20 cm beträgt und die kürzeste Abmessung des Lochs zwischen 1 cm und 6 cm beträgt.

## Revendications

1. Support orthétique dynamique (1,100) pour la gestion d'une fasciite plantaire, comprenant une chaussette (3, 110) présentant une partie semelle (7, 120) formée dans une matière élastique (111), dans lequel la partie semelle (7,120) est formée de telle sorte que, quand le support est porté, des lignes de tension apparaissent dans la partie semelle (7,120) qui amènent un pied du porteur vers une position fermée, afin de soulager les symptômes de la fasciite plantaire,
**caractérisé en ce que** la partie semelle (7, 120) comprend une pince (34, 130) formée en fronçant ou plissant la matière au niveau de la partie semelle (7, 120) et en joignant la matière froncée ou plissée, ou en retirant une partie de matière de la partie semelle (7, 120) formant ainsi un trou défini à travers la partie semelle (7, 120), et en joignant des bords du trou, la pince (34, 130) formant une indentation (33, 140) dans la partie semelle (7, 120).

2. Support orthétique selon la revendication 1, dans lequel la partie semelle (7, 120) comprend une section de talon (9, 121), une section de plante de pied (122), et une section de voûte (123) ; la partie semelle (7, 120) étant formée de telle sorte que, quand le support est porté, la matière de la section de voûte soit étirée davantage que la matière de la section de talon (9, 121) ou que la matière de la section de plante de pied (122).

3. Support orthétique selon la revendication 1 ou la revendication 2, dans lequel l'indentation (33, 140) est une indentation sensiblement conique qui, quand elle est étirée pour épouser le pied du porteur, fait apparaître des lignes de tension qui servent à fermer le pied du porteur.

4. Support orthétique selon la revendication 2 ou la revendication 3, dans lequel la pince (34, 130) s'étend sensiblement centralement le long de la partie semelle (7, 120), facultativement dans lequel la pince (34, 130) s'étend entre la section de plante de pied (122) de la partie semelle (7, 120) et la section de talon (9, 121) de la partie semelle.

5. Support orthétique selon l'une quelconque des revendications précédentes comprenant au moins un panneau de renforcement élastique (15, 17, 19, 21, 151, 152, 153, 154) fixé à la partie semelle (7, 120) de la chaussette afin de, quand le support est porté, amener un pied du porteur vers une position fermée.

6. Support orthétique selon la revendication 5, dans lequel le, ou chaque, panneau de renforcement élastique (15, 17, 19, 21, 151, 152, 153, 154), quand le support est porté, exerce une force contractile ou ligne de tension diagonalement en travers d'au moins une partie de la partie semelle (7, 120).

7. Support orthétique selon la revendication 5 ou la revendication 6, dans lequel :
a) un panneau de renforcement élastique (15, 151) s'étend diagonalement vers l'arrière en travers d'au moins une partie de la partie semelle (7, 120) depuis un côté droit de la section de plante de pied (122) de la partie semelle (7, 120), facultativement, dans lequel le panneau de renforcement élastique (15, 151) s'étend depuis le côté droit de la section de plante de pied (122) jusqu'à une position sur la partie semelle (7, 120) qui est sensiblement équidistante depuis un côté droit et un côté gauche de la partie semelle (7, 120) ;
b) un panneau de renforcement élastique (17, 152) s'étend diagonalement vers l'arrière en travers d'au moins une partie de la partie semelle (7, 120) depuis un côté gauche de la section de plante de pied (122) de la partie semelle (7, 120), facultativement dans lequel le panneau de renforcement élastique (17, 152) s'étend depuis le côté gauche de la section de plante de pied (122) jusqu'à une position sur la partie semelle (7, 120) qui est sensiblement équidistante depuis un côté droit et un côté gauche de la partie semelle (7, 120) ;
c) un panneau de renforcement élastique (21, 154) s'étend diagonalement vers l'avant en travers d'au moins une partie de la partie semelle (7, 120) de la chaussette (3, 110) depuis un côté gauche d'une section de talon (9, 121) de la partie semelle (7, 120), facultativement dans lequel le panneau de renforcement élastique (21, 154) s'étend depuis le côté gauche de la section de talon (9, 121) de la partie semelle (7, 120) de la chaussette (3, 110) jusqu'à une position sur la partie semelle (7, 120) qui est sensiblement équidistante depuis un côté droit et un côté gauche de la partie semelle (7, 120) ; et/ou
d) un panneau de renforcement élastique (19, 153) s'étend diagonalement vers l'avant en travers d'au moins une partie de la partie semelle (7, 120) depuis un côté droit d'une section de talon (9, 121) de la partie semelle (7, 120), facultativement dans lequel le panneau de renforcement élastique (19, 153) s'étend depuis le côté droit de la section de talon (9, 121) de la partie semelle (7, 120) jusqu'à une position sur la partie semelle (7, 120) qui est sensiblement équidistante depuis un côté droit et un côté gauche de la partie semelle (7, 120).

8. Support orthétique selon l'une quelconque des revendications 5 à 7, dans lequel le, ou chaque, panneau de renforcement élastique (15, 17, 19, 21, 151, 152, 153, 154) sur la partie semelle (7, 120) présente une partie qui s'étend jusqu'à une section de voûte (123) sur la partie semelle (7, 120), de préférence jusqu'à l'indentation (33, 140) dans la partie semelle (7, 120).

9. Support orthétique selon l'une quelconque des revendications précédentes comprenant au moins un panneau de renforcement élastique (23, 25) qui s'étend en travers d'une partie de talon (9) de la chaussette (3, 110), et/ou comprenant au moins un panneau de renforcement élastique (27) fixé à la chaussette de telle sorte que, quand le support est porté, la dorsiflexion d'un pied du porteur soit encouragée.

10. Procédé de réalisation d'un support orthétique (1, 100) pour la gestion d'une fasciite plantaire, comprenant les étapes de formation d'une chaussette (3, 110) à partir d'une matière élastique (111) destinée à épouser un pied du porteur, **caractérisé en ce que** le procédé comprend la formation d'une pince (34, 130) dans une partie de semelle (7, 120) de la chaussette (3, 110) de telle sorte qu'une partie de la partie semelle (7, 120) soit indentée par rapport au reste de la chaussette (3, 110), dans lequel la pince (34, 130) est formée en fronçant ou plissant la matière au niveau de la partie semelle (7, 120) de la chaussette (3, 110) et en joignant la matière froncée ou plissée pour former la pince (34, 130), ou en retirant une partie de matière de la partie semelle (7, 120) formant ainsi un trou défini à travers la partie semelle, et en joignant des bords du trou pour former la pince (34, 130).

11. Procédé selon la revendication 10, comprenant l'étape de fixation d'au moins un panneau de renforcement élastique (15, 17, 19, 21, 151, 152, 153, 154) à la partie semelle (7, 120) de la chaussette (3, 110), de préférence avant l'étape de formation de la pince (34, 130), facultativement, dans lequel le, ou chaque, panneau de renforcement élastique (15, 17, 19, 21, 151, 152, 153, 154) est fixé à la partie semelle (7, 120) de la chaussette (3, 110) dans une orientation diagonale afin d'exercer une force contractile ou ligne de tension diagonalement en travers d'au moins une partie de la partie semelle (7, 120) de la chaussette (3, 110) quand le support est porté.

12. Procédé selon la revendication 11, dans lequel :
a) un panneau de renforcement élastique (15, 151) fixé à la chaussette (3, 110) s'étend diagonalement vers l'arrière en travers d'au moins une partie de la partie semelle (7, 120) depuis un côté droit de la section de plante de pied (122) de la partie semelle (7, 120), facultativement dans lequel le panneau de renforcement élastique (15, 151) est fixé de telle sorte qu'il s'étende depuis le côté droit de la section de plante de pied (122) jusqu'à une position sur la partie semelle (7, 120) qui est sensiblement équidistante depuis un côté droit et un côté gauche de la partie semelle (7, 120) ;
b) un panneau de renforcement élastique (17, 152) fixé à la chaussette (3, 110) s'étend diagonalement vers l'arrière en travers d'au moins une partie de la partie semelle (7, 120) depuis un côté gauche de la section de plante de pied (122) de la partie semelle (7, 120), facultativement dans lequel le panneau de renforcement élastique (17, 152) est fixé de telle sorte qu'il s'étende depuis le côté gauche de la section de plante de pied (122) jusqu'à une position sur la partie semelle (7, 120) qui est sensiblement équidistante depuis un côté droit et un côté gauche de la partie semelle (7, 120) ;
c) un panneau de renforcement élastique (21, 154) fixé à la chaussette (3, 110) s'étend diagonalement vers l'avant en travers d'au moins une partie de la partie semelle (7, 120) de la chaussette (3, 110) depuis un côté gauche d'une section de talon (9, 121) de la partie semelle (7, 120), facultativement dans lequel le panneau de renforcement élastique (21, 154) est fixé de telle sorte qu'il s'étende depuis le côté gauche de la section de talon (9, 121) de la partie semelle (7, 20 120) de la chaussette (3, 110) jusqu'à une position sur la partie semelle (7, 120) qui est sensiblement équidistante depuis un côté droit et un côté gauche de la partie semelle (7, 120) ; et/ou
d) un panneau de renforcement élastique (19, 153) fixé à la chaussette (3, 110) s'étend diagonalement vers l'avant en travers d'au moins une partie de la partie semelle (7, 120) depuis un côté droit d'une section de talon (9, 121) de la partie semelle (7, 121), facultativement dans lequel le panneau de renforcement élastique (19, 153) est fixé de telle sorte qu'il s'étende depuis le côté droit de la section de talon (9, 121) de la partie semelle (7, 120) jusqu'à une position sur la partie semelle (7, 120) qui est sensiblement équidistante depuis un côté droit et un côté gauche de la partie semelle (7, 120).

13. Procédé selon la revendication 11, dans lequel le, ou chaque, panneau de renforcement élastique (15, 17, 19, 21, 151, 152, 153, 154) est fixé à la partie semelle (7, 120) de la chaussette (3, 110) de manière à avoir une partie, ou extrémité, qui s'étend jusqu'à une section de voûte (123) sur la partie semelle (7, 120), de préférence jusqu'à la pince (34, 130) dans la partie semelle (7, 120).

14. Procédé selon l'une quelconque des revendications 11 à 13 comprenant les étapes consistant à fixer au moins un panneau de renforcement élastique (23, 25) en travers d'une partie de talon (9, 121)) au niveau d'une partie arrière de la chaussette (3, 110), et/ou à fixer un panneau de renforcement élastique favorisant la dorsiflexion (27) qui, quand le support est porté, encourage la dorsiflexion d'un pied du porteur, facultativement dans lequel le panneau de renforcement favorisant la dorsiflexion (27) est fixé à la chaussette (3, 110) de telle sorte qu'il s'étende depuis une section de tibia de la chaussette jusqu'à une section de haut de pied de la chaussette (3, 110).

15. Procédé selon la revendication 10 et l'une quelconque des revendications précédentes dépendant de la revendication 10, dans lequel le trou est de forme sensiblement ovale, l'axe long de l'ovale s'étendant dans un sens talon vers orteils, facultativement dans lequel la plus longue dimension du trou est comprise entre 3 cm et 20 cm, et la plus courte dimension du trou est comprise entre 1 cm et 6 cm.
